# EUROPEAN PATENT SPECIFICATION

(11) **EP 3 785 603 B1**
(45) Date of publication and mention of the grant of the patent: **02.08.2023**
(21) Application number: 19792203.2
(22) Date of filing: 25.04.2019
(51) Int. Cl.: G06V 10/46, G06V 30/18, A61B 3/14, G06F 18/24

(54) **MACHINE LEARNING-BASED FUNDUS IMAGE DETECTION METHOD, APPARATUS, AND SYSTEM**
VERFAHREN, VORRICHTUNG UND SYSTEM ZUR ERFASSUNG DES AUGENHINTERGRUNDES AUF DER BASIS VON MASCHINELLEM LERNEN
PROCÉDÉ, APPAREIL ET SYSTÈME DE DÉTECTION PAR RÉTINOPHOTOGRAPHIE SUR LA BASE DE L'APPRENTISSAGE AUTOMATIQUE

(30) Priority: 26.04.2018 CN 201810387484
(43) Date of publication of application: 03.03.2021
(73) Proprietor: Shanghai Eaglevision Medical Technology Co., Ltd., Shanghai 200030 (CN)
(72) Inventor: XIONG, Jianhao, Shanghai 200030 (CN); ZHAO, Xin, Shanghai 200030 (CN); MA, Yongpei, Shanghai 200030 (CN); LI, Shulei, Shanghai 200030 (CN); HE, Chao, Shanghai 200030 (CN); ZHANG, Dalei, Shanghai 200030 (CN)
(74) Representative: Germain Maureau
(86) International application number: PCT/CN2019/084210
(87) International publication number: WO 2019/206209

(56) References cited:
- WO-A1-2016/032397
- CN-A- 105 411 525
- CN-A- 108 577 803
- CN-A- 108 596 895
- US-A1- 2012 257 164
- US-A1- 2014 314 288
- US-A1- 2014 314 288
- US-B1- 9 008 391
- US-B1- 9 462 945
- ZOU XIAOCHUN ET AL: "Learning-Based Visual Saliency Model for Detecting Diabetic Macular Edema in Retinal Image", COMPUTATIONAL INTELLIGENCE AND NEUROSCIENCE, vol. 2016, 31 January 2016 (2016-01-31), pages 1-10, XP055883352, US ISSN: 1687-5265, DOI: 10.1155/2016/7496735 Retrieved from the Internet: URL:https://www.ncbi.nlm.nih.gov/pmc/artic les/PMC4738732/pdf/CIN2016-7496735.pdf> [retrieved on 2022-01-25]

## Description

This application claims priority to Chinese Patent Application No. 201810387484.8, entitled "Method, Apparatus and System for Detecting Fundus Image Based on Machine Learning" filed on April 26, 2018.

### Field of the Invention

The present invention relates to the field of medical image recognition technology, and specifically to a method, apparatus and system for detecting a fundus image based on machine learning.

### Background of the Invention

In recent years, machine learning has been widely used in the medical field. Especially, the machine learning technology represented by deep learning has been widely concerned in the medical imaging field. For example, in the aspect of fundus image detection, the deep learning technology can detect a certain feature of a fundus image more accurately. For example, a deep learning model is trained using a large number of samples having a feature of macular holes, and macular hole detection is performed on the fundus image by using the trained model. These technologies are often limited to the detection of a single feature or a few associated features, and cannot accurately detect other features. However, the eye is a very fine and complex organ in the human body and contains a wide variety of features, and the features are often greatly different. Therefore, the detection results are difficult to converge with the use of the existing detection technologies, and the detection results are inaccurate accordingly. Or, a model is trained for detecting each feature or features, which not only requires a large number of samples, but also sharply increases the amount of calculation in the presence of numerous features, resulting in a decrease in the detection efficiency. Document US 2014/314288 (2014-10-23) discloses a method for detecting an abnormal fundus image by detecting candidate blood vessel regions and classifying them as to whether they include bright and red lesions, and combining the results to determine the degree of severity of a lesion in the fundus image.

Therefore, how to quickly and accurately detect a fundus image has become a technical problem to be solved urgently.

### Summary of the Invention

In a first aspect, provided is a method for detecting a fundus image as defined by claim 1.

In a further aspect, provided is an electronic device as defined by claim 8.

In a further aspect, provided is a computer storage medium as defined by claim 9.

In a further aspect, provided is a computer program product as defined by claim 10.

In a further aspect, provided is a system according to claim 11.

### Brief Description of the Drawings

In order to describe the technical solutions in the specific embodiments of the present invention or in the prior art more clearly, the following briefly introduces the accompanying drawings required for describing the specific embodiments or the prior art. Apparently, the accompanying drawings in the following description show only some embodiments of the present invention, and those of ordinary skilled in the art may still derive other drawings from these accompanying drawings without creative efforts.
Fig. 1 is a flowchart of a method for detecting a fundus image based on machine learning;
Fig. 2 is a flowchart of another method for detecting a fundus image based on machine learning;
Fig. 3 is a schematic diagram of an apparatus for detecting a fundus image based on machine learning;
Fig. 4 is a schematic diagram of a preferred apparatus for detecting a fundus image;
Fig. 5 is a schematic diagram of an electronic device for executing the method for detecting a fundus image;
Fig. 6 is a schematic diagram of a system for detecting a fundus image based on machine learning.

### Detailed Description of Embodiments

The following clearly and completely describes the technical solutions of the present invention with reference to the accompanying drawings.

In the description of the present invention, it should be noted that the terms "first", "second" and "third" are only for the sake of description, and cannot be understood as indicating or implying the relative importance. Further, the technical features involved in the present invention described below may be combined with each other as long as they do not constitute a conflict with each other.

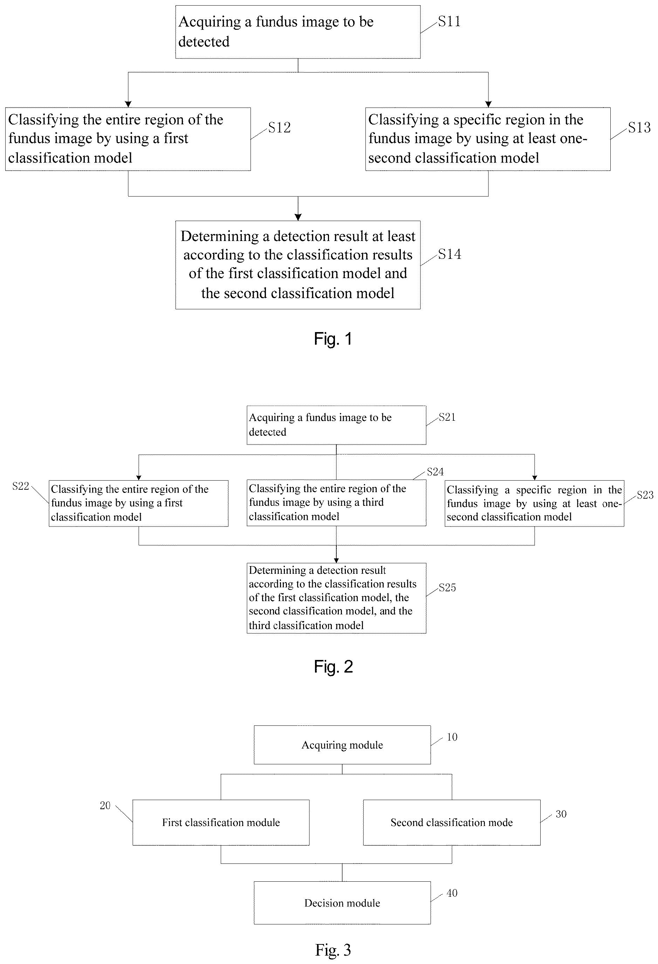

An example of the present invention provides a method for detecting a fundus image based on machine learning, which can be executed by a computer or a server. As shown in Fig. 1, the method includes the following steps:
S11: acquiring a fundus image to be detected. The image is a fundus retina image shot by an eye detecting device for diagnosing an eye lesion.
S12: classifying the entire region of the fundus image by using a first classification model to determine whether the fundus image contains any first feature. The first features are image features having high saliency. The so-called saliency may be weighed by factors such as chromatic aberration, contrast, and grayscale, or the size of the occupied region. For example, if the entire region of the fundus image has a region whose color is greatly different from the normal fundus color, and the ratio of the region is greater than a certain preset value, the region is taken as the first feature.

Specifically, the first features may include a large area of abnormal tissue or structure within the fundus, large spots within the fundus, and the like, such as image features related to lesions such as leopard fundus, fundus white spots, and fundus laser spots.

The first features are detected using a machine learning algorithm. Before the process of the model, the classification model should be trained using a large number of fundus image samples having various first features, so that the classification model has a certain capability of classification. The first classification model may be a single-classification model or a multi-classification model. If it is a single classification model, the output result is two classes, that is, containing or not containing the first features; if it is a multi-classification model, the output result is multiple classes, that is, not containing any first feature or the class of the contained first feature.

S13: classifying a specific region in the fundus image by using at least one-second classification model to determine whether the fundus image contains any second feature, wherein the saliency of the first features is greater than that of the second features. Steps S12 and S13 are preferably executed synchronously or executed in any order.

The second feature should be interpreted as a detailed feature, and the saliency of the first feature is greater than that of the second feature. For example, the chromatic aberration, contrast, grayscale, or area of the second features is smaller than that of the first features. In fact, since the first classification model carries out a global classification for the image, the first classification model and the second classification model both detect the second features, but the first classification model is less sensitive to the second features, while the second classification model is more sensitive.

The second features are in the specific region. The specific region includes at least one of an optic disc region, a macular region, a blood vessel region and a retinal region, and may also be one region or a plurality of regions within a set range. For example, the specific region is an optic disc region, and the second features include a specific feature such as shape abnormality of the optic disc, color abnormality of the optic disc, or abnormality of the optic nerve; alternatively, the specific region is macular, and the second feature includes a specific feature such as macular structural abnormality, or macular shape abnormality; alternatively, the specific region is a blood vessel region, and the second features include a specific feature such as color abnormality of the blood vessel, trend abnormality of the blood vessel, shape abnormality of the central vein, or shape abnormality of the branch vein; alternatively, the specific region is a retinal region, and the second features include small abnormal points such as color abnormal points, irregular points, or reduction of the retinal region. Besides, the second features may also include features of other details in the fundus, such as vascular grains.

The second features are processed using a machine learning algorithm. Before the process, the corresponding classification model should be trained using a large number of fundus image samples having various second features, so that the classification model has a certain capability of classification.

In the invention a plurality of second classification models are used for parallel classification detection for different specific regions, and each of the second classification models independently outputs a classification result. Specifically, for example, three-second classification models are used: the classification model A is used for an optic disc region and detects whether the optic disc region contains specific features related to the optic disc, for example, features of various optic disc lesions such as papilledema, papillitis, and optic atrophy; the classification model B is used for a macular region and detects whether the macular region contains specific features related to the macula, for example, features of various macular lesions such as macular holes, macular edema, and cartographic atrophy of the macular region; and the classification model C is used for a blood vessel region and detects whether the blood vessel region contains specific features related to the blood vessel, for example, features of various blood vessel lesions such as vitreous hemorrhage, choroidal hemangioma, central vein occlusion, and branch vein occlusion.

To reduce the amount of calculation, the second classification model may be configured to output binary classification results to indicate the presence or absence of the second features of the fundus image. To improve the accuracy of detection, the second classification models may be configured to output multi-classification results to indicate that the fundus image does not contain any second feature, or the specific class of the contained second features. In actual application, the output of the multi-classification results or the single-classification results may be determined according to whether the various specific classes obtained by the second classification model conflict.

S14: determining a analysis result at least according to the classification results of the first classification model and the second classification model. This step can completely follow the classification results of the two classification models, that is, directly output their classification results, or judge the classification results of the two classification models to obtain the final analysis result. The so-called judgment refers to determine a comprehensive result according to the combination of the classification results output by the first classification model and the second classification model. The final result may be inconsistent with the classification results output by the two classification models.

In the example the classification results of the first classification model and the second classification model are classified through a machine learning algorithm, and the classification result is used in the final result.

The classification result output by the classification model is generally a numerical value, specifically confidence information or probability expressed by 0-1. In this solution, the value output by the classification model can be used as the final result, the value can also be further judged, and a corresponding result is determined based on the value.

Before applied to the judgment, a large number of sample data should be used to train the decision module so that it has a certain classification ability. The sample data should include information about whether it has any first feature and second feature and its corresponding label or include information about the specific classes of the first features and the second features and its corresponding label.

The entire region of the fundus image to be detected is detected by the first features having high saliency, and the specific region of the fundus image is simultaneously detected by the second features having low saliency so that the two classification models do not interfere with each other and work clearly, and each region can be accurately classified to determine whether it contains relevant features; and the result is finally obtained by judgment in combination with the classification results of the two features to improve the accuracy of the final result, thereby achieving simultaneous analysis of features of multiple categories and multiple saliencies, with higher efficiency.

Since the fundus photos taken by an image shooter are very different in quality, the photos are often overexposed, gray, and blurry, which greatly increases the difficulty of machine learning judgment. As an alternative embodiment, the quality of images is analysed to screen qualified images, which further ensures the accuracy of image analysis. In a specific embodiment, the fundus image may be subjected to any one or any combination analysis of stain/ bright spot, exposure, sharpness, light leakage, and local shadow.

Specifically, regarding the stain/ bright spot detection, a plurality of images to be detected are weighted and averaged to obtain an average image, and whether the average image has pixels exceeding a preset brightness range is then judged; when the average image has pixels exceeding the preset brightness range, it is confirmed that the image to be detected has stains/ bright spots. The detection of stains or bright spots can be completed.

Regarding the light leakage detection, the image to be detected is binarized to obtain a preset region in the image; a mask based on the boundary of the preset region is generated; the mask is fused with the image to be detected; the average color brightness of the image after fusion is calculated and compared with a preset color brightness threshold the degree of light leakage of the image to be detected is confirmed according to the comparison result. When the degree of light leakage is greater than a preset value, it can be confirmed that the fundus image has light leakage.

Regarding the local shadow detection, a histogram of any color channel in the image to be detected is counted; the number of pixels smaller than a preset pixel value is counted; whether the number of pixels smaller than the preset pixel value is less than a preset number is judged; and when the number of pixels smaller than the preset pixel value is less than the preset number, it is confirmed that the image to be detected has a local shadow.

Regarding the sharpness detection, a high-frequency component of the image to be detected is extracted; an amount of information of the high-frequency component is calculated, and the sharpness of the image to be detected is confirmed based on the amount of information of the high-frequency component.

Regarding the exposure detection, the image to be detected is converted into a gray image; a root mean square of a histogram of the gray image is counted and the exposure of the image to be detected is confirmed based on the root mean square.

When the fundus image has the above quality problems, the detection result of the image may be affected and may be inaccurate. Therefore, to ensure the detection accuracy of the image, the image having the above quality defects may be removed before the classification operation.

In practical applications, some features in the fundus image, especially some features having less saliency may not exist in the specific region of the fundus image, and the detection only for the features having less saliency in the specific region may result in missing detection. To improve the comprehensiveness and accuracy of the detection, the embodiment of the present invention provides a method for detecting a fundus image. As shown in Fig. 2, the method includes the following steps:
S21: acquiring a fundus image to be detected.
S22: classifying the entire region of the fundus image by using a first classification model to determine whether the fundus image contains any first feature. For details, reference may be made to the description of the first feature detection in step S12 of the above embodiment.
S23: classifying a specific region in the fundus image by using at least one-second classification model to determine whether the fundus image contains any second feature. For details, reference may be made to the description of the second feature detection in step S13 of the above embodiment.
S24: classifying the entire region of the fundus image by using a third classification model to determine whether the fundus image contains any third feature, wherein the saliency of the third features is smaller than that of the second features. The third features are finer features than the second features, for example, a distributed lesion feature such as a minor lesion of the fundus. Steps S22, S23, and S24 are preferably executed synchronously or executed in any order.

To reduce the amount of calculation, the third classification model may be configured to output a binary classification result to indicate the presence or absence of the third features of the fundus image. To improve the accuracy of detection, the third classification model may be configured to output a multi-classification result to indicate that the fundus image does not contain any third feature, or the specific class of the contained third features.

S25: determining a detection result according to the classification results of the first classification model, the second classification model, and the third classification model. Regarding the description of the detection result in step S14 of the foregoing embodiment, the classification result of the third features is further added here, so that the detection result is more accurate.

When the decision model is used to determine the final detection result, the input data of the decision model is whether it has any first feature, second feature, and third feature, or the specific classes of the first features, the second features, and the third features.

The above various classification models are implemented by a convolutional neural network. The basic units of the convolutional neural network include convolutional layers, activation function (ReLu) layers, and pooling layers. The convolutional layers screen-specific image features, the activation function layers nonlinearly process the screened features by using a ReLu activation function, and the pooling layers extract the strongest information at different locations using max pooling. Batch normalization may be used during information extraction to improve the capacity of the network while preventing gradient dispersion in the process of training the network. Through a plurality of such basic units, the features in the fundus image can be extracted and finally output by fully connected layers and an output layer (softmax).

The number of network layers of each module varies from 15 to 100 layers based on the classes of fundus features that need to be detected. Specifically, the convolutional neural network may be implemented as the following structure: input layer - C1 - BN1 - R1 - P1 - C2 - BN2 - R2 - P2 - C3 - BN3 - R3 - P3 - C4 - BN4 - R4 - P4 - C5- BN5 - R5 - P5 - FC1 - FC2 - SoftMax. An image of a certain size is located on the input layer, C represents a convolutional layer (e.g., C1, C2, C3, C4, C5), BN represents a batch normalization layer (e.g., BN1, BN2, BN3, BN4, BN5), R represents a function activation layer (e.g., R1, R2, R3, R4, R5), P represents a pooling layer (e.g., P1, P2, P3, P4, P5), the fully connected layers include FC1 and FC2, and SoftMax provides an output. The convolutional neural network used in the present embodiment is not limited to the structure of the convolutional neural network described above, and other neural network structures satisfying the present embodiment are also applicable.

Since the saliency of the first features is greater than the saliency of the second feature and third feature set, the sizes of hidden layers of the neural network can be changed according to the saliency of the features, the hidden layers being from the input to the output. Specifically, small hidden layers are used for the features having high saliency, while large hidden layers are used for the features having low saliency. The maximum hidden layer of the convolutional network for the second feature and third feature set having low saliency is larger than the maximum hidden layer of the convolutional network for the first features.

Specifically, when the first features are detected, since the features have high saliency, the maximum hidden layer of the network is required to be small, for example, less than 200×200, facilitating feature extraction. For the second feature or third feature set having low saliency, the output of the maximum hidden layer should be kept large, for example, more than 300×300, ensuring that fine fundus sub-features such as small exudation points and bleeding points can be extracted. The output of the hidden layer with a maximum size is determined by the image input layer, the convolutional layers, and the pooling layers together, and is implemented in various ways, and details are not described herein again.

An embodiment of the present invention provides an apparatus for detecting a fundus image as defined by claim 8. Fig. 3 shows an apparatus corresponding to the example of Figure 1. This apparatus includes an acquiring module 10, configured to acquire a fundus image to be detected a first classification module 20, configured to classify the entire region of the fundus image to determine whether the fundus image contains any first feature at least one-second classification model 30, configured to classify a specific region in the fundus image to determine whether the fundus image contains any second feature, wherein the saliency of the first features is greater than that of the second features; and a decision module 40, configured to determine a detection result at least according to the classification results of the first classification model and the second classification model.

In an alternative embodiment, in the presence of a plurality of specific regions and a plurality of second classification models 30, the plurality of second classification models are respectively configured to classify different specific regions and output second classification results, the second classification results being used to indicate whether the fundus image contains second features related to the specific regions.

In an alternative embodiment, the first classification model and the second classification model are both multi-classification models, and the classification results thereof are used to indicate whether the fundus image contains the first features and the second features, and the specific classes of the first features and the second features.

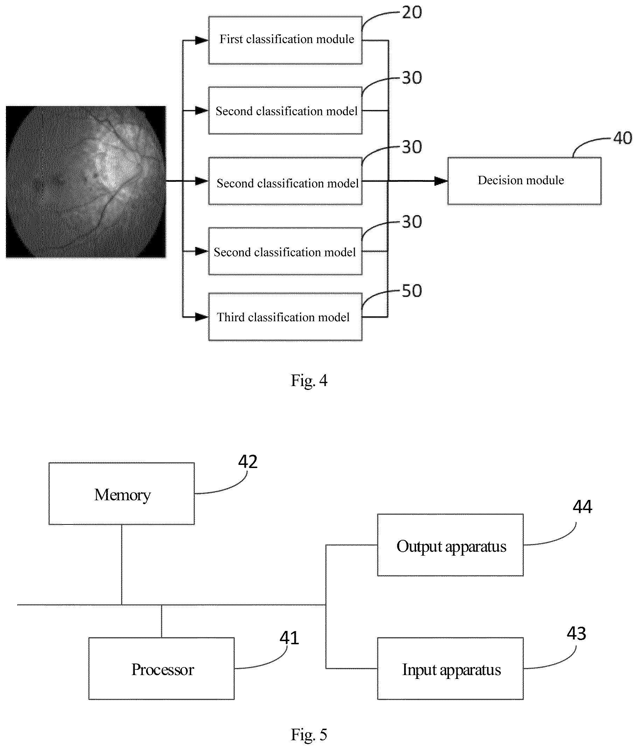

In the embodiment, as shown in Fig. 4, the detecting apparatus further includes:
A third classification model 50, configured to classify the entire region of the fundus image to determine whether the fundus image contains any third feature, wherein the saliency of the third features is smaller than that of the second features;
The decision module 40 determines the detection result according to the classification results of the first classification model 20, the second classification model 30, and the third classification model 50.

In an alternative embodiment, the third classification model is a multi-classification model, and the classification result thereof is used to indicate whether the fundus image contains any third feature and the specific class of the third features.

The specific region includes at least one of an optic disc region, a macular region, a blood vessel region, and a retinal region.

In an alternative embodiment, the first features, the second features, and the third features are all fundus lesion features.

An electronic device may be a server or a terminal. As shown in Fig. 5, a controller is included, the controller includes one or more processors 41 and a memory 42, and one processor 43 is taken as an example in Fig. 5.

The electronic device may further include an input apparatus 43 and an output apparatus 44.

The processor 41, the memory 42, the input apparatus 43, and the output apparatus 44 may be connected by a bus or other means, exemplified by a bus in Fig. 4.

The processor 41 may be a Central Processing Unit (CPU). The processor may be other general-purpose processors, such as Digital Signal Processor (DSP), Application Specific Integrated Circuit (ASIC), Field-Programmable Gate Array (FPGA) or other programmable logic devices, such as discrete gate or transistor logic device, a discrete hardware component or other chips, or a combination of the various chips. The general-purpose processor may be a microprocessor or any conventional processor, etc.

As a non-transitory computer-readable storage medium, the memory 42 can be used for storing non-transitory software programs, non-transitory computer-executable programs, and modules. The processor 41 runs the non-transitory software programs, instructions, and modules stored in the memory 42 to execute various function applications of the server and data processing, that is, to implement the method for detecting a fundus image in the above method embodiment.

The memory 42 may include a program storage region and a data storage region. The program storage region may store an operating system, and an application program required by at least one function. The data storage region may store data created according to the use of processing apparatuses for the server. Besides, memory 42 may include a high-speed random access memory, and may also include a non-transitory memory, for example, at least one magnetic disk storage device, a flash memory, or other non-transitory solid-state storage devices. In some embodiments, the memory 42 may alternatively include memories remotely disposed relative to the processor 41, and these remote memories may be connected to a network connecting apparatus through a network. Examples of the network include, but are not limited to, the Internet, an intranet, a local area network, a mobile communications network, or a combination thereof.

The input apparatus 43 may receive input digit or character information, and generate a key signal input related to the user setting and function control of the processing apparatuses of the server. The output apparatus 44 may include a display device such as a display screen.

One or more modules are stored in the memory 42, and when executed by one or more processors 41, implement the method as shown in Fig. 1 or 2.

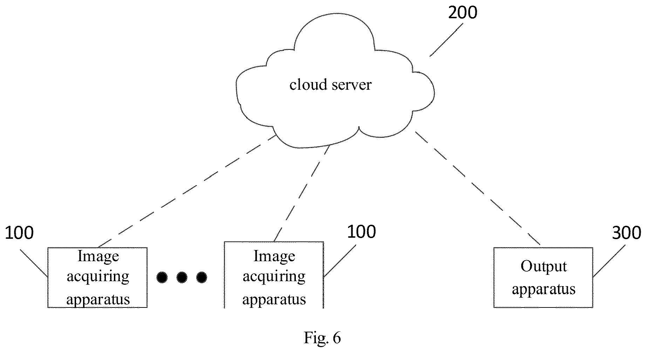

An embodiment of the present invention further provides a system for analysing a fundus image based on machine learning. As shown in Fig. 6, the system includes an image acquiring apparatus 100, configured to acquire a fundus image. In the present embodiment, the image acquiring apparatus may be plural. Specifically, for example, the image acquiring apparatus 100 is a fundus shooting device in each hospital, or a fundus shooting device of an individual user. In the present embodiment, the fundus analysis system further includes a cloud server 200. An apparatus for analysing a fundus image for executing the method for analysing a fundus image is provided in the cloud server 200. The cloud server 200 communicates with the image acquiring apparatus 100, for example, in the form of wireless communication, or wired communication. The fundus image acquired by the image acquiring apparatus 100 is uploaded to the cloud server 200, an electronic device executes the method for analysing a fundus image to obtain a analysis result, and an output apparatus outputs the analysis result. Specifically, the output apparatus 300 may be a display device, or a printing device for printing in the form of a report, or a user terminal device, such as a mobile phone, tablet, or personal computer.

A person skilled in the art should understand that the embodiments of the present invention may be provided as a method, a system, or a computer program product. Therefore, the present invention may be in the form of a full hardware embodiment, a full software embodiment, or an embodiment combining software and hardware. Besides, the present invention may be in the form of a computer program product implemented on one or more computer available storage media (including but not limited to a disk memory, a CD-ROM, an optical memory, and the like) including computer available program codes.

The present invention is described regarding flow diagrams and/or block diagrams of the method, equipment (system), and the computer program product in the embodiments of the present invention. It should be understood that computer program instructions can implement each process and/or block in the flowcharts and/or block diagrams and a combination of processes and/or blocks in the flowcharts and/or block diagrams. These computer program instructions may be provided to a general-purpose computer, a dedicated computer, an embedded processor, or a processor of other programmable data processing equipment to generate a machine, so that a device configured to implement functions specified in one or more processes in the flowcharts and/or one or more blocks in the block diagrams is generated by using instructions executed by the general-purpose computer or the processor of other programmable data processing equipments.

These computer program instructions may also be stored in a computer-readable memory that can guide a computer or other programmable data processing equipment to work in a specific manner, so that the instructions stored in the computer-readable memory generate a product including an instruction device, where the instruction device implements functions specified in one or more processes in the flowcharts and/or one or more blocks in the block diagrams.

These computer program instructions may also be loaded into a computer or other programmable data processing equipment, so that a series of operation steps are performed on the computer or other programmable data processing device to generate processing implemented by a computer, and instructions executed on the computer or other programmable data processing equipment provide steps for implementing functions specified in one or more processes in the flowcharts and/or one or more blocks in the block diagrams.

## Claims

1. A method for detecting a fundus image based on machine learning, comprising:
acquiring a fundus image to be detected;
classifying an entire region of the fundus image by using a first classification model to determine whether the fundus image contains a first feature or features, wherein the first feature or features indicate abnormal tissue or structure in the entire region of the fundus image;
classifying, in parallel, one or more specific regions in the fundus image by using a second classification model per specific region to determine whether the fundus image contains a second feature or features, wherein the second feature or features indicate abnormal tissue or structure in the one or more specific regions of the fundus image, the specific region including at least one of an optic disc region, a macular region, a blood vessel region, a retinal region, and one or more regions within a set range, wherein a saliency of the first feature or features is greater than that of the second feature or features, the saliency is weighted by one or more of the following factors: chromatic aberration, contrast, grayscale, or a size of an occupied region;
classifying the entire region of the fundus image by using a third classification model to determine whether the fundus image contains a third feature or features, wherein the saliency of the third features is smaller than that of the second features; and
determining a detection result indicating the probability that the fundus image includes one or more of optic disc lesions, macular lesions and blood vessel lesions according to the classification results of the first classification model, the second classification model and the third classification model;
and wherein the first classification model, the second classification model and the third classification model are respectively implemented as a convolutional neural network, and a maximum hidden layers of the convolutional neural network for the second features and third features are larger than a maximum hidden layer of the convolutional neural network for the first features.

2. The method according to claim 1, wherein the specific region and the second classification model are respectively plural, and in the step of classifying a specific region in the fundus image by using at least one-second classification model, different second classification models are respectively used to classify different specific regions and output second classification results, the second classification results being used to indicate whether the fundus image contains second features related to the specific regions.

3. The method according to claim 1 or 2, wherein the first classification model and the second classification model are both multi-classification models, and the classification results thereof are used to indicate whether the fundus image contains the first features and the second features, and the specific classes of the first features and the second features.

4. The method according to any one of claims 1-3, wherein the specific region comprises at least one of an optic disc region, a macular region, a blood vessel region, and a retinal region.

5. The method according to any one of claims 1-4, wherein the first features and the second features are both fundus lesion features.

6. The method according to claim 1, wherein the third features are fundus lesion features.

7. The method according to claim 1, wherein the third classification model is a multi-classification model, and the classification result thereof is used to indicate whether the fundus image contains any third feature and the specific class of the third features.

8. An electronic device, comprising at least one processor; and a memory connected to the at least one processor by communication; wherein the memory stores instructions executable by the one processor, and the instructions are executed by the at least one processor to cause the at least one processor to implement the method for analysing a fundus image according to any one of claims 1-7.

9. A computer storage medium, storing instructions thereon that, when running on a computer, cause the computer to implement the method for analysing a fundus image according to any one of claims 1-7.

10. A computer program product comprising instructions, when running on a computer, causing the computer to implement the method for detecting a fundus image according to any one of claims 1-7.

11. A system for analysing a fundus image based on machine learning, comprising:
an image acquiring apparatus, configured to acquire a fundus image;
the device according to claim 8, communicating with the image acquiring apparatus to analyse the fundus image; and
an output apparatus, communicating with an apparatus for analysing a fundus image to output a analysis result of the fundus image.

## Patentansprüche

1. Verfahren zum Detektieren eines Fundusbildes basierend auf maschinellem Lernen, das Folgendes umfasst:
Erfassen eines zu detektierenden Fundusbildes;
Klassifizieren einer gesamten Region des Fundusbildes unter Verwendung eines ersten Klassifizierungsmodells, um zu bestimmen, ob das Fundusbild ein erstes Merkmal oder erste Merkmale enthält, wobei das erste Merkmal oder die ersten Merkmale auf abnormales Gewebe oder eine abnormale Struktur in der gesamten Region des Fundusbildes hinweisen;
paralleles Klassifizieren einer oder mehrerer spezifischer Regionen im Fundusbild unter Verwendung eines zweiten Klassifizierungsmodells pro spezifischer Region, um zu bestimmen, ob das Fundusbild ein zweites Merkmal oder zweite Merkmale enthält, wobei das zweite Merkmal oder die zweiten Merkmale auf abnormales Gewebe oder eine abnormale Struktur in der einen oder den mehreren spezifischen Regionen des Fundusbildes hinweisen, wobei die spezifische Region mindestens eine aus einer Sehnervenregion, einer Makularegion, einer Blutgefäßregion, einer Netzhautregion und einer oder mehreren Regionen innerhalb eines festgelegten Bereichs umfasst, wobei eine Erkennbarkeit des ersten Merkmals oder der ersten Merkmale größer ist als die des zweiten Merkmals oder der zweiten Merkmale, wobei die Erkennbarkeit durch einen oder mehrere der folgenden Faktoren gewichtet wird: Chromatische Aberration, Kontrast, Graustufen oder die Größe einer belegten Region;
Klassifizieren der gesamten Region des Fundusbildes unter Verwendung eines dritten Klassifizierungsmodells, um zu bestimmen, ob das Fundusbild ein drittes Merkmal oder dritte Merkmale enthält, wobei die Erkennbarkeit der dritten Merkmale kleiner ist als die der zweiten Merkmale; und
Bestimmen eines Detektionsergebnisses, das die Wahrscheinlichkeit angibt, dass das Fundusbild eine oder mehrere Läsionen des Sehnervs, Makulaläsionen und Blutgefäßläsionen gemäß den Klassifizierungsergebnissen des ersten Klassifizierungsmodells, des zweiten Klassifizierungsmodells und des dritten Klassifizierungsmodells enthält;
und wobei das erste Klassifizierungsmodell, das zweite Klassifizierungsmodell und das dritte Klassifizierungsmodell jeweils als neuronales Faltungsnetzwerk implementiert sind und eine maximale verborgene Schicht des neuronalen Faltungsnetzwerks für die zweiten Merkmale und die dritten Merkmale größer ist als eine maximale verborgene Schicht des neuronalen Faltungsnetzwerks für die ersten Merkmale.

2. Verfahren nach Anspruch 1, wobei die spezifische Region und das zweite Klassifizierungsmodell jeweils mehrere sind und im Schritt des Klassifizierens einer spezifischen Region im Fundusbild unter Verwendung von mindestens einem zweiten Klassifizierungsmodell unterschiedliche zweite Klassifizierungsmodelle jeweils verwendet werden, um verschiedene spezifische Regionen zu klassifizieren und zweite Klassifizierungsergebnisse auszugeben, wobei die zweiten Klassifizierungsergebnisse verwendet werden, um anzuzeigen, ob das Fundusbild zweite Merkmale enthält, die mit den spezifischen Regionen im Zusammenhang stehen.

3. Verfahren nach Anspruch 1 oder 2, wobei das erste Klassifizierungsmodell und das zweite Klassifizierungsmodell beide Mehrfachklassifizierungsmodelle sind und die Klassifizierungsergebnisse davon verwendet werden, um anzuzeigen, ob das Fundusbild die ersten Merkmale und die zweiten Merkmale und die spezifischen Klassen der ersten Merkmale und der zweiten Merkmale enthält.

4. Verfahren nach einem der Ansprüche 1-3, wobei die spezifische Region mindestens eine aus einer Sehnervenregion, einer Makularegion, einer Blutgefäßregion und einer Netzhautregion umfasst.

5. Verfahren nach einem der Ansprüche 1-4, wobei die ersten Merkmale und die zweiten Merkmale beide Fundusläsionsmerkmale sind.

6. Verfahren nach Anspruch 1, wobei die dritten Merkmale Fundusläsionsmerkmale sind.

7. Verfahren nach Anspruch 1, wobei das dritte Klassifizierungsmodell ein Mehrfachklassifizierungsmodell ist und das Klassifizierungsergebnis davon verwendet wird, um anzuzeigen, ob das Fundusbild ein drittes Merkmal und die spezifische Klasse der dritten Merkmale enthält.

8. Elektronische Vorrichtung, die mindestens einen Prozessor und einen Speicher, der durch Kommunikation mit dem mindestens einen Prozessor verbunden ist, umfasst; wobei der Speicher Anweisungen speichert, die von dem einen Prozessor ausführbar sind, und die Anweisungen von dem mindestens einen Prozessor ausgeführt werden, um den mindestens einen Prozessor dazu zu veranlassen, das Verfahren zum Analysieren eines Fundusbildes nach einem der Ansprüche 1-7 zu implementieren.

9. Computerspeichermedium, auf dem Anweisungen gespeichert sind, die, wenn sie auf einem Computer ausgeführt werden, den Computer dazu veranlassen, das Verfahren zum Analysieren eines Fundusbildes nach einem der Ansprüche 1-7 zu implementieren.

10. Computerprogrammprodukt, das Anweisungen umfasst, die, wenn sie auf einem Computer ausgeführt werden, den Computer dazu veranlassen, das Verfahren zum Detektieren eines Fundusbildes nach einem der Ansprüche 1-7 zu implementieren.

11. System zum Analysieren eines Fundusbildes basierend auf maschinellem Lernen, Folgendes umfassend:
ein Bilderfassungsgerät, das zum Erfassen eines Fundusbildes konfiguriert ist;
wobei die Vorrichtung nach Anspruch 8 mit dem Bilderfassungsgerät kommuniziert, um das Fundusbild zu analysieren; und
ein Ausgabegerät, das mit einem Gerät zum Analysieren eines Fundusbildes kommuniziert, um ein Analyseergebnis des Fundusbildes auszugeben.

## Revendications

1. Procédé de détection d'une image de fond d'oeil sur la base d'un apprentissage automatique, comprenant :
l'acquisition d'une image de fond d'oeil à détecter ;
la classification d'une région entière de l'image de fond d'oeil en utilisant un premier modèle de classification pour déterminer si l'image de fond d'oeil contient une ou des premières caractéristiques, dans laquelle la ou les premières caractéristiques indiquent un tissu ou une structure anormal dans la région entière de l'image de fond d'oeil ;
la classification, en parallèle, d'une ou de plusieurs régions spécifiques dans l'image de fond d'oeil en utilisant un deuxième modèle de classification par région spécifique pour déterminer si l'image de fond d'oeil contient une ou des deuxièmes caractéristiques, dans laquelle la ou les deuxièmes caractéristiques indiquent un tissu ou une structure anormal dans la ou les plusieurs régions spécifiques de l'image de fond d'oeil, la région spécifique incluant au moins l'une d'une région de disque optique, d'une région maculaire, d'une région de vaisseau sanguin, d'une région rétinienne et d'une ou de plusieurs régions dans une plage fixée, où une saillance de la ou des premières caractéristiques est supérieure à celle de la ou des deuxièmes caractéristiques, la saillance est pondérée par un ou plusieurs des facteurs suivants : une aberration chromatique, un contraste, une échelle de gris ou une taille d'une région occupée ;
la classification de la région entière de l'image de fond d'oeil en utilisant un troisième modèle de classification pour déterminer si l'image de fond d'oeil contient une ou des troisièmes caractéristiques, dans laquelle la saillance des troisièmes caractéristiques est inférieure à celle des deuxièmes caractéristiques ; et
la détermination d'un résultat de détection indiquant la probabilité que l'image de fond d'oeil comprenne une ou plusieurs de lésions de disque optique, de lésions maculaires et de lésions de vaisseau sanguin selon les résultats de classification du premier modèle de classification, du deuxième modèle de classification et du troisième modèle de classification ;
et dans lequel le premier modèle de classification, le deuxième modèle de classification et le troisième modèle de classification sont respectivement mis en oeuvre comme un réseau neuronal convolutif, et des couches cachées maximales du réseau neuronal convolutif pour les deuxièmes caractéristiques et les troisièmes caractéristiques sont plus grandes qu'une couche cachée maximale du réseau neuronal convolutif pour les premières caractéristiques.

2. Procédé selon la revendication 1, dans lequel la région spécifique et le deuxième modèle de classification sont respectivement multiples, et dans l'étape de classification d'une région spécifique dans l'image de fond d'oeil en utilisant au moins un deuxième modèle de classification, des deuxièmes modèles de classification différents sont respectivement utilisés pour classer différentes régions spécifiques et fournir en sortie des deuxièmes résultats de classification, les deuxièmes résultats de classification étant utilisés pour indiquer si l'image de fond d'oeil contient des deuxièmes caractéristiques liées aux régions spécifiques.

3. Procédé selon la revendication 1 ou 2, dans lequel le premier modèle de classification et le deuxième modèle de classification sont tous deux des modèles de multi-classification, et leurs résultats de classification sont utilisés pour indiquer si l'image de fond d'oeil contient les premières caractéristiques et les deuxièmes caractéristiques, et les classes spécifiques des premières caractéristiques et des deuxièmes caractéristiques.

4. Procédé selon l'une quelconque des revendications 1 à 3, dans lequel la région spécifique comprend au moins l'une d'une région de disque optique, d'une région maculaire, d'une région de vaisseau sanguin et d'une région rétinienne.

5. Procédé selon l'une quelconque des revendications 1 à 4, dans lequel les premières caractéristiques et les deuxièmes caractéristiques sont toutes deux des caractéristiques de lésion du fond d'oeil.

6. Procédé selon la revendication 1, dans lequel les troisièmes caractéristiques sont des caractéristiques de lésion du fond d'oeil.

7. Procédé selon la revendication 1, dans lequel le troisième modèle de classification est un modèle de multi-classification, et son résultat de classification est utilisé pour indiquer si l'image de fond d'oeil contient une troisième caractéristique quelconque et la classe spécifique des troisièmes caractéristiques.

8. Dispositif électronique, comprenant au moins un processeur ; et une mémoire reliée à l'au moins un processeur par communication ; où la mémoire stocke des instructions exécutables par le processeur, et les instructions sont exécutées par l'au moins un processeur pour amener l'au moins un processeur à mettre en oeuvre le procédé d'analyse d'une image de fond d'oeil selon l'une quelconque des revendications 1 à 7.

9. Support de stockage informatique, sur lequel sont stockées des instructions qui, lorsqu'elles s'exécutent sur un ordinateur, amènent l'ordinateur à mettre en oeuvre le procédé d'analyse d'une image de fond d'oeil selon l'une quelconque des revendications 1 à 7.

10. Produit de programme informatique comprenant des instructions, lorsqu'elles s'exécutent sur un ordinateur, amenant l'ordinateur à mettre en oeuvre le procédé de détection d'une image de fond d'oeil selon l'une quelconque des revendications 1 à 7.

11. Système d'analyse d'une image de fond d'oeil sur la base d'un apprentissage automatique, comprenant :
un appareil d'acquisition d'image, configuré pour acquérir une image de fond d'oeil ;
le dispositif selon la revendication 8, communiquant avec l'appareil d'acquisition d'image pour analyser l'image de fond d'oeil ; et
un appareil de sortie, communiquant avec un appareil pour analyser une image de fond d'oeil pour fournir en sortie un résultat d'analyse de l'image de fond d'oeil.
